# EUROPEAN PATENT SPECIFICATION

(11) **EP 4 609 949 B1**
(45) Date of publication and mention of the grant of the patent: **04.11.2026**
(21) Application number: 24159833.3
(22) Date of filing: 27.02.2024
(51) Int. Cl.: B01L 3/00, B01L 7/00, G01N 21/64, C12Q 1/686

(54) **A MICROFLUIDICS CHIP FOR PERFORMING A BIOCHEMICAL REACTION**
MIKROFLUIDIKCHIP ZUR DURCHFÜHRUNG EINER BIOCHEMISCHEN REAKTION
PUCE MICROFLUIDIQUE POUR EFFECTUER UNE RÉACTION BIOCHIMIQUE

(43) Date of publication of application: 03.09.2025
(73) Proprietor: Imec VZW, 3001 Leuven (BE)
(72) Inventor: Fauvart, Maarten, 3060 Bertem (BE); Lagae, Liesbet, 3000 Leuven (BE); Rottenberg, Xavier, 3010 Kessel-Lo (BE); Stakenborg, Tim, 3001 Heverlee (BE); Taher, Ahmed, 3001 Heverlee (BE); Yurt, Abdulkadir, 3001 Heverlee (BE)
(74) Representative: Roth, Sebastian

(56) References cited:
- WO-A1-2015/047190
- WO-A1-2021/040624
- WO-A2-03/035229
- US-A1- 2023 381 772

## Description

### TECHNICAL FIELD

The present disclosure relates to microfluidics chips and methods for performing individually addressable biochemical reactions, for example, thermally induced biochemical reactions like a polymerase chain reaction (PCR). For example, a microfluidics chip for performing such a biochemical reaction is provided. The chip may include means for heating and optically reading out cavities of the chip. In addition, a microfluidics system comprising the microfluidics chip, and a method for operating the microfluidics chip are provided.

### BACKGROUND

As a prominent example of a (thermal) biochemical reaction, digital Polymerase chain reaction (PCR) is a technique used for detecting and quantifying a specific nucleic acid sequence of interest in a sample - also referred to as reaction mixture in this disclosure - with an option for post-processing through extraction after amplification. Digital PCR, like other biochemical reactions, typically involves compartmentalizing the reaction mixture before thermal cycling. The compartmentalization usually involves generating a plurality of droplets of the reaction mixture in an immiscible phase, e.g. oil.

However, generating droplets is prone to error and requires highly skilled operators. Moreover, isolating and extracting the amplified material from the droplets is also quite difficult.

WO 2015/047190 A discloses a microfluidic device comprising: a plurality of wells, each well having an inlet and an outlet, where the inlets are in fluid communication with one or more entry channels and the outlets are in fluid communication with one or more exit channels, wherein said outlet is connected to the exit channel via an outlet connecting channel and said inlet is connected to the entry channel via an inlet connecting channel.

WO 03/035229 A2 discloses an apparatus comprising a substrate with one or more assay stations or wells and channels arranged in a manner to facilitate the flow of fluids through the apparatus and designed to provide for isolation-medium sealing of the assay stations.

WO 2021/040624 A1 discloses a microfluidic device, and methods of manufacturing microfluidic devices.

US 2023/0381772 A1 discloses a gene amplification chip, an apparatus for gene amplification, and an apparatus for bio-particle analysis.

### SUMMARY

The solutions provided in the present disclosure are based further on the following considerations of the inventors.

The use of physical cavities can offer several advantages, e.g., stability of the compartments and immobilization of the reaction mixture. The cavities may be implemented on glass, which allows optically monitoring the cavities during the PCR or any other biochemical reaction, and also allows a subsequent in situ analysis, for example, to determine the number of copies implemented in the digital PCR, or to perform alternative quantification methods induced by heat, like high-resolution melting.

The larger the number of the cavities, the larger the dynamic range and sensitivity will be. Hence a large area solution is preferred. Conventional solutions typically use micro-wells on glass or polymer, but these have limitations, including significant sample loss during loading, restricted handling after loading, and inefficient thermal control for processes like fast thermal cycling. The conventional solutions offer also no possibility to integrate heaters. These solutions thus typically use non-integrated heating, which makes thermally induced biochemical reactions like PCR very slow. Contact heating such as Peltier elements will also not heat fast, because of the low thermal conductivity of the glass. The conventional methods do also not allow to address parts of the chip for example to address different samples. The conventional solutions further offer only limited possibilities for a large area addressable and real time readout, and do not facilitate in situ cavity readout.

In view of the above, an improved cavity-based solution for biochemical reactions is desired. An objective is to provide a solution that offers accurate microfluidic loading of a reaction mixture into cavities, good thermal control, and the possibility of in situ cavity readout. An objective is also to enable recovery of the contents of individual cavities after performing the biochemical reaction. Other objectives are limited sample (reaction mixture) loss during cavity loading, a large area solution, and the possibility to automate the sample loading procedure.

These and other objectives are achieved by the solutions described in the independent claims. Advantageous implementations are described in the dependent claims.

A first aspect of this disclosure is a microfluidics chip for performing a biochemical reaction in a reaction mixture, the microfluidics chip comprising at least one sample unit wherein the sample unit comprises: a plurality of cavities configured to receive the reaction mixture, wherein each cavity has an inlet and an outlet; a first fluid channel for transporting the reaction mixture to the plurality of cavities, wherein the first fluid channel is fluidically connected to the inlet of each cavity; a second fluid channel for transporting oil, wherein the second fluid channel is connected to the outlet of each cavity; wherein the outlet of each cavity comprises a capillary barrier for the reaction mixture, the capillary barrier comprising a part of the cavity, the part of the cavity having a smaller diameter than the rest of the cavity, wherein the capillary barrier of the outlet of each cavity is configured such that under normal pressure conditions and operation of the chip during receiving the reaction mixture by each cavity, surface-tension forces retain the reaction mixture within the cavity and prevent the reaction mixture from passing from the cavity to the second fluid channel, and that gas is allowed to pass from the cavity to the second fluid channel. The chip further comprises an optical readout layer, wherein the optical readout layer comprises an optical detector array, and at least one of a lenslet array and a spectral-filter array. The optical detector array comprises a plurality of detection pixels, wherein each cavity is aligned with one detection pixel or with one group of detection pixels, wherein the detection pixel or group of detection pixels is configured to detect light from that cavity, and the optical detector array is configured to detect light from the reaction mixture in each cavity individually.

The reaction mixture may be suitable for performing a thermal (i.e., a thermally induced) biochemical reaction. For example, the reaction mixture may be a PCR reaction mixture that is suitable to perform a PCR or loop-mediated isothermal amplification (LAMP) reaction mixture that is suitable to perform a LAMP. Such a reaction mixture can be a water-based solution buffered around a pH of 8 (e.g., a pH in a range of 7-9), containing one or more of template DNA, oligonucleotide primers, DNA polymerase, nucleotides (dNTPs) and magnesium ion cofactors.

The cavities of the chip, once filled with the reaction mixture, can be heated to enable, for instance, a thermally induced amplification of the content of the cavity, in order to receive reaction products. The cavities of the chip are preferably oriented substantially perpendicular to a flow or transport direction of the reaction mixture or oil, respectively, in the first and the second fluid channel. The orientation of the cavities is preferably vertical with respect to the plane of the chip. For instance, a plurality of vertical cavities may be arranged in an array of rows and or columns, wherein the vertical direction is perpendicular to the plane spanned by the array. This orientation of the cavities has the advantage of efficient loading of the reaction mixture into the cavities. The first fluid channel and the plurality of cavities may be configured such that after the reaction mixture is introduced into the first fluid channel, the cavities nearer to the inlet of the first fluid channel are filled first with the reaction mixture, and the reaction mixture overflows to cavities subsequently further away from the inlet of the fluid channel, after the cavities nearer to the inlet of the first fluid channel are filled completely.

The reaction mixture may be prevented from passing from the cavities to the second fluid channel by a mechanical-valve-free structure of the outlet of each cavity. The mechanical-valve-free structure is a structure that does not require mechanical force or mechanical elements for opening or closing the outlet to the reaction mixture. The mechanical-valve-free structure is configured to control the flow of the reaction mixture, in particular to prevent the reaction mixture from passing to the second fluid channel, without using traditional moving parts. The mechanical-valve-free structure may rely on material properties and/or design, like pore size and/or surface tension, to block the movement of the reaction mixture through the outlet. This significantly reduces loss of the reaction mixture (sample loss) during the loading into the cavities. For instance, the capillary effect may be employed. The mechanical-valve-free structure may be a capillary valve that is based only on capillarity and surface tension rather than mechanical parts. For example, the outlet structure could be designed such that the reaction mixture is prevented to pass during normal loading into the cavities due to the capillary effect, but may be able to pass if a sufficiently high force is applied to overcome the capillary effect. Thus, the second fluid channel may further be suitable for transporting the reaction mixture. For instance, the reaction product could be extracted from the cavities to the second fluid channel by applying a sufficient force. The reaction product could then be used for subsequent further analysis such as for example sequencing.

The chip of the first aspect may achieve some or all of the objectives mentioned above. The chip enables simple and robust microfluidic loading of the reaction mixture into the cavities via the first fluid channel. With the chip, the recovery of the contents of individual cavities after the biochemical reaction is possible. The chip has further the advantage of limited sample loss during the reaction mixture loading. It also provides the possibility for a large area solution (e.g., the number of cavities can be high and scaled), and the possibility to automate the loading procedure.

A capillary barrier is an easy way to ensure that the reaction mixture is not able to pass (on its own, for example, only due to the gravitational force) into the second fluid channel, when it has been loaded into the cavities.

In an implementation of the chip, the capillary barrier comprises a part of the cavity, the part of the cavity having a smaller diameter than the rest of the cavity, for example, having a diameter smaller than 50 µm.

The optical readout layer enables an efficient readout of the reaction mixture. In addition, the possibility to illuminate and/or optically excite the reaction mixture may be provided. Illumination and optical read out (detection) could be made from the same side of the cavities, e.g., could be implemented in one of the substrates. The illumination and optical read out could be implemented on both/opposite sides as well, e.g., using each of the two substrates. This may facilitate scaling up to larger arrays and smaller pitch sizes. The optical detector array may comprise TFTs or may be a TFT array. The optical detector array may comprise spectralfilters, which can be interference and/or absorptive filters e.g. band-pass filters such as optical color filters or notch filters, to detect multiple fluorescent dies associated with the reaction mixture.

The alignment between the filter pixels and the detection pixels can be one-to-one or group-to-group, but also a not fully aligned configuration is possible.

A precise optical readout of the cavities is thus enabled. Each detection pixel or group of detection pixels may be configured to exclusively readout one cavity. A pattern of the filter and/or detection pixels may be repeated for each cavity. This enables sensitive detection and absolute quantification of the targeted nucleic acids.

Thereby, the analysis of a specific cavity content is enabled. In situ cavity readout is possible.

Notably, the "chip" in this disclosure can also be a "device" in general. It is not necessary, for example, that all the elements of the chip described above are integrated into a package, like it is the case for many electronic chips. The chip can be a cartridge and/or can be based on glass.

In an implementation of the chip, the first fluid channel is further for transporting oil.

This has the advantage that after the loading of the reaction mixture into the cavities, the inlets of the cavities can be sealed by filling the first fluid channel with oil. For instance, no leakage of the reaction mixture is then anymore possible anymore or "cross-talk" between cavities. The same is achieved by the second fluid channel being suitable to transport oil. For instance, by filling the second fluid channel with oil after the loading of the reaction mixture into the cavities, the outlets of the cavities can be sealed as well.

In an implementation of the chip, the chip comprises a plurality of sample units wherein each sample unit comprises an individual first fluid channel. The first fluid channel of different sample units can be configured to load different reaction mixtures. Thus, different sample units can be configured to perform different biochemical reactions. It is an advantage that different biochemical reactions can be performed in parallel.

In an implementation of the chip comprising a plurality of sample units, the chip comprises an individual second fluid channel such that oil for sealing can be filled at a different time from the sealing of other sample units. In an implementation of the chip, some or all of the second fluid channels of the sample units form a common second fluid channel such that oil for sealing can be filled at the same time for some or all of the sample units on the chip.

In an implementation of the chip, the chip is for performing a thermal biochemical reaction like a PCR in the reaction mixture, the chip further comprising: one or more heating elements arranged and configured to heat the reaction mixture; and/or one or more photo-thermal elements arranged and configured to heat the reaction mixture when being illuminated.

In this way, with the heating elements, a good thermal control over the reaction mixture in the cavities is achieved. Uniform heating across multiple cavities may be achieved. For instance, the heating elements may be arranged adjacent to the first fluid channel, for example, between the first fluid channel and a substrate bordering the first fluid channel. The heating elements and/or the photo-thermal elements may be addressed (operated or illuminated) individually.

Different sample units corresponding to different samples may be provided on the chip. Each sample unit may have an individually addressable heating element so that samples can be cycled independently. As an advantage, each sample unit can be configured to perform different biochemical reactions that require different heating profiles and/or different numbers of heating cycles.

Notably, the chip of the first aspect is suitable for performing a thermal biochemical reaction (e.g., PCR) even without integrated heating or photo-thermal elements or other integrated heating source. For instance, in operation together with an external heating source. However, if the heating source is integrated into the chip, the temperature can be more efficiently and precisely provided and controlled.

In an implementation of the chip, the part of the cavity has surface modification such that the hydrophobicity of the part is configured to prevent the reaction mixture to pass through.

In an implementation of the chip, the part of the cavity has surface modification and a smaller diameter than the rest of the cavity such that the hydrophobicity of the part is configured to prevent the reaction mixture to pass through.

Hydrophobicity tuning may be used for the capillary barrier. The diameter of smaller than 50 µm could, for example, be only locally applied. Preferably, the diameter of said part of the cavity is not less than 1 µm, such that it has less impact on the transparency of the chip.

In an implementation of the chip, the plurality of cavities is an array of cavities; and/or the plurality of cavities is arranged between the first fluid channel and the second fluid channel.

In an implementation of the chip, each cavity is designed to hold a volume of the reaction mixture in a range 0.1-100 nl.

The total volume of the reaction mixture that can be held by the chip, or by one unit area on the chip, is then calculated by the volume of each cavity times the number of cavities. There may be in the order of thousands, or tens of thousands, or even hundreds of thousands of cavities. As a specific example, a total volume of 15 µl may be distributed over 20000 cavities of the chip, having a capacity of 0.75 nl per each cavity. The chip thus offers the advantage of very small cavities and a large array. With this, single molecule loading into the cavities may be enabled. Optical detection of the presence of the molecule(s) can be performed.

In an implementation of the chip, the chip further comprises two transparent substrates, wherein the assembly of the first fluid channel, the second fluid channel, and the plurality of cavities is arranged between the two transparent substrates.

The substrates may be glass substrates. The substrates may be panels, and may cover a large area. Hence multiple samples can be processed in parallel.

Each sample may be associated with its own microfluidic loading structure, i.e. first fluid channel, that loads the corresponding reaction mixture to one sample unit on the chip. In an implementation of the chip, each sample unit may have at least 1000, more preferably at least 10000 cavities. For example, if 8 samples are loaded, 8 sample units each comprising, for instance, 20000 cavities may be provided.

Further, optical access from the top and the bottom to the fluid channels and/or cavities may be provided.

It is also possible, that only one substrate is transparent, to provide optical access from one side of the chip. The other substrate could, for example, have a reflecting surface, in order to allow more efficient readout or illumination.

In an implementation of the chip, the cavities and at least one of the first fluid channel and the second fluid channel are transparent, so as to allow optical access.

For instance, optical access to the reaction mixture in the cavities can be enabled. In this way, the reaction mixture may be optically sensed (for read out or for analysis) and/or may be illuminated. This is useful to optically monitor the thermal biochemical reaction continuously, or only read out the reaction product at the end.

In an implementation of the chip, the chip may be a cartridge.

A second aspect of this disclosure is a microfluidics system comprising: a chip according to the first aspect or any implementation thereof; a reaction mixture reservoir, which is fluidically connectable to the first fluid channel of the chip; and an oil reservoir, which is fluidically connectable to the first fluid channel and to the second fluid channel of the chip.

Any feature of the first aspect may be as correspondingly described in the second aspect.

The system may comprise more than one such chip. Each chip in case of multiple chips, e.g. cartridges, may be connected to common reservoirs.

In an implementation of the microfluidics system, the microfluidics system comprises: multiple chips, wherein each chip is configured according to the first aspect or any implementation thereof; wherein a reaction mixture reservoir is fluidically connectable to the respective first fluid channel of each chip and an oil reservoir is fluidically connectable to the respective first fluid channel and to the respective second fluid channel of each chip; and a controller configured to control the microfluidics system to perform a biochemical reaction in one or more of the multiple chips.

In this way, several biochemical reactions can be multiplexed in different chips, for instance, biochemical reactions on different samples or different biochemical reactions. Each chip may comprise one or a plurality of sample units, each comprising a cavity array. In the example of plurality of sample units, the multiple sample units may be connected to multiple reaction mixture reservoirs and oil reservoirs, respectively. That is, one per each sample unit. The oil reservoir could potentially be shared among the sample units.

In an implementation of the microfluidics system, the microfluidics system further comprises a controller configured to control the microfluidics system to perform the method of the third aspect described below.

In particular, the controller may be configured to control an automatic loading procedure of the reaction mixture into the cavities. The controller may select the type of liquid -reaction mixture or oil - to flow in and/or fill the fluid channels. The controller may also be configured to control heaters, for example, to perform PCR involving heat cycles or to address individual samples. The controller may also be configured to control an optical readout using the optical detector array. For example, it may receive and prove a digital signal received from each of a plurality of optical sensors of the optical detector array. In an implementation, the controller further comprises an image processing unit configured to convert the digital sensor signals to quantifiable data for the PCR. The optical detector array may be designed to obtain an analog signal (e.g., if it includes an avalanche photodiode (APD) array). In this case, an external DC circuit of the chip may be configured to digitize the analog signal for downstream data processing by the controller or on a computer. Alternatively, the optical detector array may contain suitable ADC circuitry, like CMOS, CCD image sensors. In this case, the optical detector array may output digital data to the controller or a computer.

An aspect of this disclosure is further a computer program comprising instructions which, when the program is executed by a controller of the above microfluidics system, cause the controller to control the microfluidics system to perform the method of the third aspect described below.

The controller may respectively comprise processing circuitry configured to perform, conduct or initiate various operations of the controller described in this disclosure. The processing circuitry may comprise hardware and/or the processing circuitry may be controlled by software. The hardware may comprise analog circuitry or digital circuitry, or both analog and digital circuitry. The digital circuitry may comprise components such as application-specific integrated circuits (ASICs), field-programmable arrays (FPGAs), digital signal processors (DSPs), or multi-purpose processors. The controller may also comprise memory circuitry, which stores one or more instruction(s) that can be executed by the processing circuitry, in particular under control of the software.

A third aspect this disclosure is a method for operating a microfluidics chip or a microfluidics system according to the above aspects and implementations, wherein the method comprises: filling the plurality of cavities of the chip with the reaction mixture via the first fluid channel; then filling the second fluid channel with oil, so as to seal the outlet of each cavity; and then filling the first fluid channel with oil, so as to seal the inlet of each cavity.

In an implementation, a pressure of the oil in the second fluid channel may be controlled. For instance, a lower pressure may be required for the oil in the second fluid channel, when extracting an analyte in a cavity to a droplet in the second fluid channel. For instance, a cavity may be locally heated, in order to nucleate a bubble in the reaction mixture in the cavity, which may push the reaction mixture into the oil. In this scenario, lower oil pressure is beneficial.

In an implementation of the method, the method further comprises, after filling the first fluid channel and the second fluid channel with oil, applying a thermal heat cycle, for example, so as to perform a PCR in the reaction mixture.

The thermal heat cycle may be applied by the one or more heating elements or the one or more photo-thermal elements described above.

The method of the third aspect achieves similar advantages as described above for the chip of the first aspect.

In summary of the above aspects and implementations, a novel design of a microfluidics device (chip) for biochemical reactions, like quantified thermally induced biochemical reactions in cavities (e.g. digital PCR) is provided. The chip may comprise transparent substrates, a microfluidic loading structure including the first fluid channel to load the reaction mixture into cavities, and may comprise patterned heating elements and detection elements or pixels. The reaction mixture may be distributed over a large quantity of the cavities (e.g., thousands or more) using either pressure driven or capillary driven flow. The cavities may be sealed using an oil, which may be added to the second fluid channel and the first fluid channel, respectively. Multiple types of reaction mixture can be loaded.

The solution of this disclosure also provides means for fast heating and cooling. The solution of this disclosure also provides means to optically readout and quantify the signal per cavity in situ and in real time, in order to do digital PCR or melting curve analysis, for example. That is a pixelized heating and optical readout may be enabled, for instance, beneficial for performing PCR. The melting analysis is another application, where such a pixelized heating and readout is beneficial. Some enzymes, e.g. ligation etc., may also work on specific temperatures (not cycling but just go to specific temperature to make it work). Moreover, the content of each cavity - e.g. a product of the biochemical reaction - can be extracted by increasing the pressure inside the cavity using a laser beam to create a bubble inside it allowing additional analysis e.g. by sequencing.

### BRIEF DESCRIPTION OF THE DRAWINGS

The above described aspects and implementations are explained in the following description of embodiments with respect to the enclosed drawings:
- FIG. 1: shows a sectional view of a chip according to this disclosure.
- FIG. 2: shows a perspective view of a chip according to this disclosure.
- FIG. 3: shows a sectional view of an exemplary chip according to this disclosure, which comprises an optical readout layer.
- FIG. 4: shows a sectional view of an exemplary chip according to this disclosure, which comprises an optical readout layer and a lenslet array.
- FIG. 5: shows an example of a layer structure for the optical readout layer.
- FIG. 6: illustrates a method of operating a chip according to this disclosure, in particular, a loading procedure.
- FIG. 7: illustrates further the method of operating the chip, in particular, an oil loading or sealing procedure.
- FIG. 8: shows a flow-diagram of a method for operating a chip according to this disclosure.
- FIG. 9: illustrates a procedure of extracting content of a cavity into the second fluid channel.
- FIG. 10: shows a microfluidics system according to this disclosure.

### DETAILED DESCRIPTION OF EMBODIMENTS

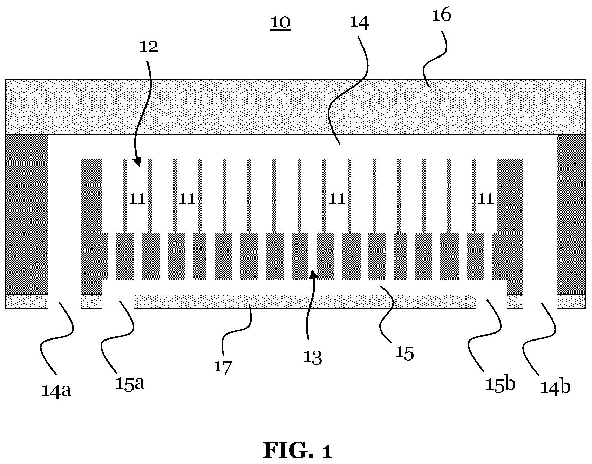

FIG. 1 shows a microfluidics chip 10 according to this disclosure. The chip 10 is usable to perform a biochemical reaction, for example a thermally induced biochemical reaction like PCR, in a reaction mixture. The reaction mixture may be loaded into the chip 10 for this purpose. The chip 10 may be a cartridge. Further, the chip 10 may be part of a microfluidics system, and may be connected to various fluid reservoirs also comprises in this system. If the chip 10 is not operated, it does not comprise the reaction mixture.

The microfluidics chip 10 comprises a plurality of cavities 11, which may be arranged in an array. The cavities 11 are configured to receive the reaction mixture. In particular, each cavity 11 is able to hold a certain amount of the reaction mixture. For instance, each cavity 11 may be designed to hold a volume of the reaction mixture in a range of a few nanoliter, for instance, 0.1-100 nl. The cavities 11 of the chip 10 can also be empty. Each cavity 11 may have a diameter of around 50 µm, and may have a depth/length of around 300-400 µm. A total area covered by cavities 11 may be in an order of 10x10 mm².

Each cavity 11 has an inlet 12 and an outlet 13, both being openings. The structure of the outlet 13 of each cavity 11 is configured to allow gas to exit the cavity 11, but prevents the reaction mixture in the cavity 11 to exit the cavity 11 through the outlet 13. This may be achieved, for instance, by implementing a capillary barrier, which may be realized by a narrow cavity portion at the outlet 13, as illustrated. The narrow cavity portion may have a diameter in a range of 10-50 µm.

The microfluidics chip 10 further comprises a first fluid channel 14 and a second fluid channel 15. The first fluid channel 14 suitable to transport the reaction mixture to the plurality of cavities 11, but may also be suitable to transport an oil. The first fluid channel 14 is fluidically connected to the inlet 12 of each cavity 11, so that the reaction mixture flowing in the first fluid channel 14 may enter the cavities 11. This is enabled by the structure of the outlet 13 being configured to pass gas from the cavity 11 to the second fluid channel 15, i.e., air can be pushed out of the cavity 11 by the reaction mixture entering it through the inlet 12. The reaction mixture itself will stop at the outlet 13 during the filling, since the structure of the outlet 13 prevents the reaction mixture from passing from the cavity 11 to the second fluid channel 15, at least if no additional force or mechanism is applied. The first fluid channel 14 may comprise an inlet 14a and an outlet 14b, which may respectively be connectable to a reservoir for, e.g., the reaction mixture. The first fluid channel 14 and the second fluid channel 15 may respectively have a width (across the flow direction) of around 30-70 µm, e.g. around 50 µm.

The second fluid channel 15 is connected to the outlet 12 of each cavity 11. The second fluid channel 15 is suitable to transport oil, wherein the oil can be used to seal the outlets 13 of the cavities 11. However, the second fluid channel 15 may also be suitable to transport the reaction mixture or another liquid. The second fluid channel 15 may comprise an inlet 15a and outlet 15b, which may be connectable to a reservoir for, e.g., the oil.

The first fluid channel 14, the cavities 11, and the second fluid channel 15 may be arranged between substrates 16 and 17. For example, the first fluid channel 14 may be formed between a first substrate 16 and the cavities 11, while the second fluid channel 15 may be formed between a second substrate 17 and the cavities 11. The substrates 16, 17 may be transparent. For instance, the substrates 16, 17 may be made of glass, or of a plastic material. The cavities 11 may be formed by glass as well, or may be formed by a structured plastic or generally dielectric material.

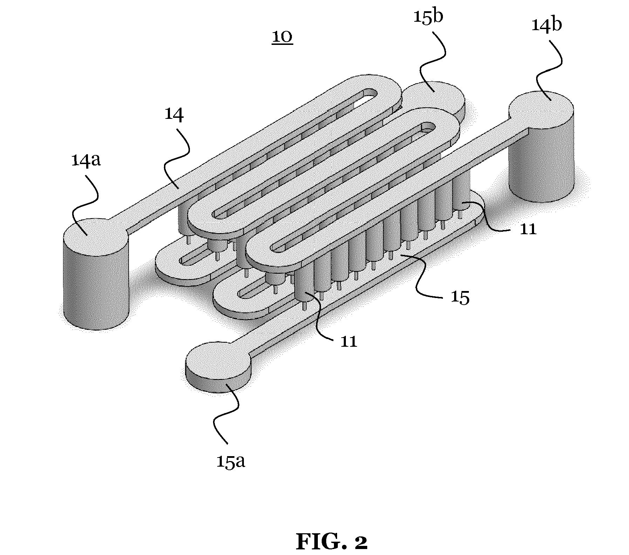

FIG. 2 shows a perspective view of an exemplary chip 10 according to this disclosure. The design of the chip 10 may be based on the chip 10 shown in FIG. 1. Same elements in FIG. 1 and FIG. 2 are labelled with the same reference numbers and may function likewise.

As can be seen in FIG. 2, the array of cavities 11 may be arranged between the first and the second fluid channel 14, 15. The fluid channels 14, 15, may respectively extend in a meandering or looped manner, so as to connect consecutively multiple rows of cavities 11 of the array. The cavities 11 may be micro-cavities implemented for instance, by glass and/or between two glass substrates 16, 17 (not shown).

In an example, the chip 10 may comprise a stack of a substrates 16, 17, wherein the cavities 11 may be formed in an inner substrate, and the first and the second fluid channel 14, 15 may be formed between the inner substrate and respectively two outer substrates 16, 17 sandwiching the inner substrate. The channels 14, 15 may be used for loading and optionally unloading the chip 10. Optionally, a patterned absorber material suitable to effect heating and heat cycling may be included in or on one of the substrates. A top glass plane may close the system. The fluid channels 14, 15 may also be used to heat or cool down the system - particularly cavities 11 holding the reaction mixture - through fluid cycling and eventually heat-reuse between cycles. The top or bottom (outer) substrates 16, 17 can optionally include Thin Film Transistors (TFTs) or similar sensors, and optionally optical filters, for enabling in situ optical readout.

The substrates can be very large panels, which may be produced at low cost and provide the possibility to include a large number of cavities 11, or to partition the chip 10 into different sample areas each including their own cavities 11 and channels 14, 15. The cavities 11 may be designed to partition the sample(s) - i.e. reaction mixture - into small well-controlled volumes, e.g., a few nanoliter each, so that each cavity may contain only a minimal amount of target species. If the target species count (e.g., number of molecules) is smaller or equal than one, this will enable digital PCR reactions where the count of positive cavities 11 will allow to quantify the amount of species.

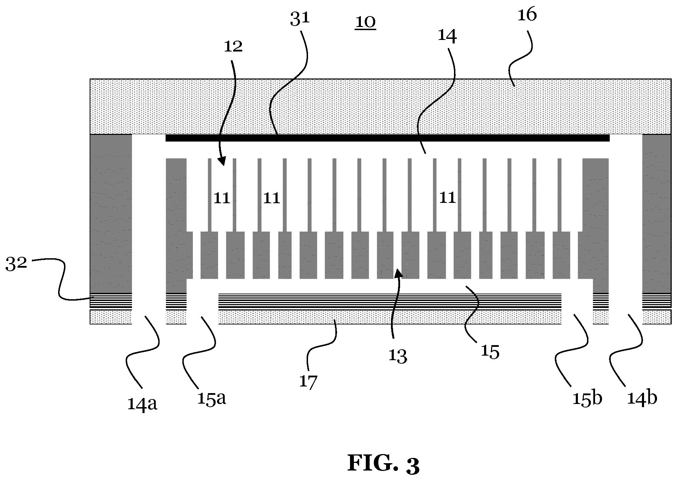

FIG. 3 shows a cross-sectional view of an exemplary chip 10 according to this disclosure. The chip 10 of FIG. 3 is based on the chip 10 shown in FIG. 1. Same elements in FIG. 1 and FIG. 3 are labelled with the same reference numbers and may function likewise.

The chip 10 of FIG. 3 may comprises one or more heating elements 31, which are arranged and configured to heat the reaction mixture. The heating elements 31 may comprise or may be connected to a metal mesh. The metal mesh may be configured as a network of local heater (metal) regions, and may allow bringing heat to the cavities 11 very fast and without having to interconnect all the cavities 11 electrically. The metal regions may be connected so that the heat gets conducted across the entire substrate 16. The metal regions could be partially optically exposed so that they heat up through optical absorbance. In this case the heating elements 31 may comprise one or more photo-thermal elements arranged and configured to heat the reaction mixture when being illuminated, for instance, by a laser. Means of illumination may be integrated in the chip 10 as well, e.g., an array of (laser) diodes. The advantageous result of the illumination is that the cavities 11 can be heated very fast. While the heating is fast, cooling may be slower. The first and/or second fluid channel 14, 15, however, may be configured to efficiently enable cool-down during operation.

Further, the chip 10 may comprise an optical readout layer, wherein the optical readout layer comprises at least an optical detector array 32. The optical readout layer may further comprise at least one of a lenslet array and a spectral-filter array. The optical detector array 32 may be pixelated, i.e., it may comprise a plurality of pixels. Each pixel of each group of a plurality of groups of pixels, may be arranged such that it is aligned with one of the cavities 11. That is, each cavity 11 may be aligned with a pixel or pixel group. At least this pixel of pixel group, which is aligned with the cavity 11, is configured to detect light stemming from that cavity 11. For instance, fluorescence light produced by the reaction mixture in the cavity 11. In an example, the optical detector array 32 may be configured to detect light from the reaction mixture in each cavity 11 individually, e.g., each pixel or pixel group may detect light from only one of the cavities 11.

The optical readout layer may be implemented in one of the substrates 16, 17. For instance, the top or bottom substrate 16, 17 can contain TFTs that are configured as the pixels, and can also contain the optical filters. In this way, an in situ integrated readout of, for instance, fluorescence in the cavities 11 becomes possible. This has the advantage of a larger area in situ read out, and a quantification across large area panels.

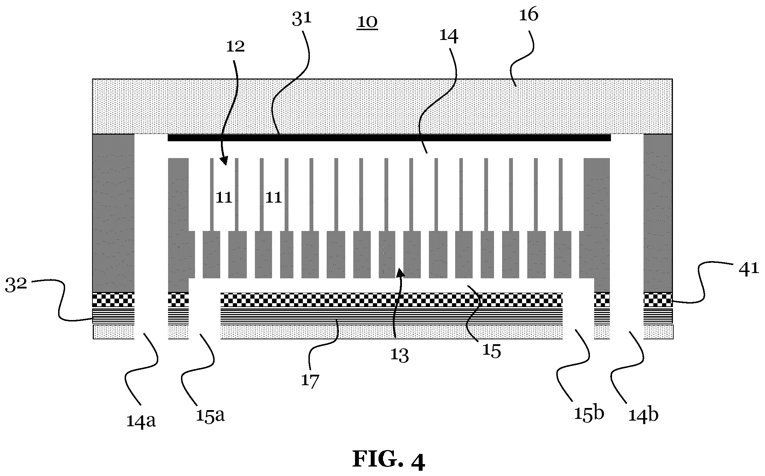

FIG. 4 shows a cross-sectional view of an exemplary chip 10 according to this disclosure. The chip 10 of FIG. 4 is based on the chips 10 shown in FIG. 1 and FIG. 3. Same elements in the FIGs. 1, 3 and 4 are labelled with the same reference numbers and may function likewise.

The chip 10 of FIG. 4 comprises the optical readout layer with the optical detector array 32 as already described with respect to FIG. 3. The optical readout layer of the chip 10 of FIG. 4 also comprises a lenslet array 41, and could further comprise a spectral-filter array. The lenslet array 41 may be arranged between the cavities 11 and the optical detector array 32. Both the lenslet array 41 and the optical detector array 32 may be patterned as layers in the substrate 17. The lenslet array 41 may comprise a plurality of lenses or micro-lenses, and may be used to focus light from the cavities 11 onto the optical detector array 32. For example, each cavity 11 may be associated with one lens configured to focus light from that cavity 11 onto the pixel or group of pixels associated with the cavity 11 in the optical detector array 32.

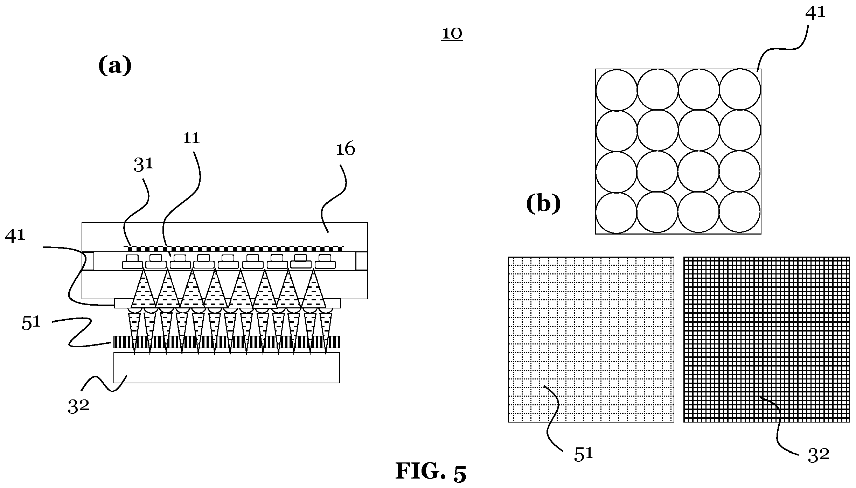

FIG. 5 shows an example of a layer structure including the optical readout layer. The layer structure may be patterned in or on one of the substrates 16, 17, specifically the substrate 17 adjacent to the second fluid channel 15. In particular, FIG. 5 shows a cross-sectional view of the layer structure (a), and shows a top view of individual layers of the optical readout layer (b).

The optical readout layer comprises the optical detector array 32, the lenslet array 41, and a spectral-filter array 51. The lenslet array 41 may be configured to focus the light onto filters or groups of filters, which are associated with detector pixels or detector pixel groups of the optical detector array 32. The optical filters may also be arranged as filter pixels or filter pixel groups, and may respectively be aligned with the detector pixels or detector pixel groups of the optical detector array 32.

In an example, each reaction cavity 11 may be aligned with one or more (e.g. a group of) detection pixels of the optical detector array 32. Further, each reaction cavity 11 may be aligned with one or more (e.g. a group of) lenses or micro-lenses of the lenslet array 41. Each of these lenses can be further aligned with one or more (e.g. a group of) filter pixels, which may simplify the lens design and increase light collection efficiency.

In a preferred implementation, each reaction cavity 11 is aligned with at least two filter pixels for filtering different wavelength(s). The intensity of photons collected in the corresponding optical detector array pixel or detector pixel group can indicate the progress of the reaction in the cavity 11. For example, the emitted light with an intensity in different wavelengths may correlate to the progress of the reaction, and thus is correlated to passed photons from certain filters.

A multicolor readout may be performed when operating the chip 10. For instance, a multicolor light source can be used, wherein the light could be used to excite fluorescent dyes of the reaction mixture in the cavities 11. The optical detector array 32 may be used to record the intensities of this fluorescent light, and a read out device (e.g., a controller) and a suitable algorithm may be used to de-convolute the fluorescent signals to correspond to the different fluorescent colors of fluorescent dies. The excitatory light may be projected as patterns on the fluorescent material, and can be de-convoluted to the intended colors. This technique has some resemblance to structured light microscopy techniques, in which multi-wavelength light patterns are projected, and afterwards de-convoluted to present multicolor images. However, in this case the intent is not to do microscopy, but to create multicolor sensor signals of the cavities 11. In a very simplistic approach, the de-convolution can comprise comparing different pixel signals of multi-wavelength patterns to understand which cavity 11 respond to which colors. Typically, one excitation color corresponds to 2-4 fluorescent dyes. A more advanced approach may use a phase control to depict dynamic light patterns, hence driving up the number of excitatory colors that can be responded to. For example, four colors could be projected as a dynamic light pattern, and de-convolution could result in 8-16 colors. The wavelengths can be projected on the optical detector (pixel) array 32 using diffractive gratings or using multiple light sources. The phase control allows to dynamically shift the light of different colors adding a layer of information that allows one to discriminate different fluorescent responses of cavities 11 from each other. It may also be possible that the optical readout layer is used to monitor the flow of liquid (e.g., reaction mixture) in the cavities 11, so that a control feedback mechanism can be provided to a fluidic controller controlling the flow of liquid, or so that a metric of reliability for an experiment may be obtained. This monitoring can avoid the necessity to omit unfilled cavities from an analysis of the biochemical reaction, for example. Alternatively, the chip 10 can be reloaded to fulfil a certain cavity filling ratio.

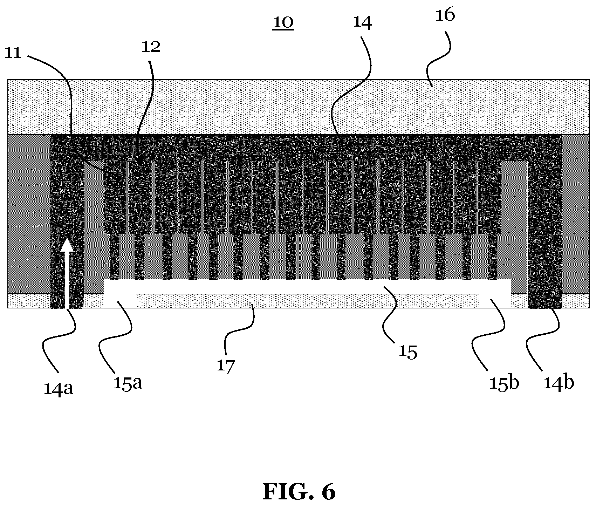

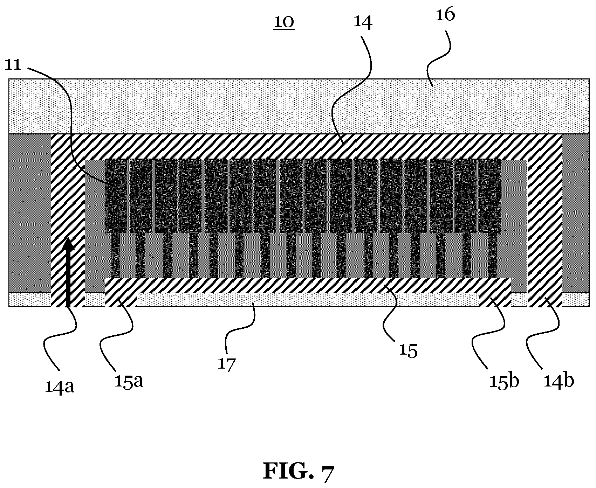

FIG. 6 and FIG. 7 illustrate a method of an operation of the chip 10, in particular, performing a loading procedure. FIG. 6 illustrates that the plurality of cavities 11 of the chip 10 may first be filled with the reaction mixture via the first fluid channel 14. The first fluid channel 14 and the cavities 11 are at some point completely filled with the reaction mixture. The outlets 13 of the cavities 11 prevent that the reaction mixture passes from the cavities 11 to the second fluid channel 15, but allow air to escape the cavities 11 during the filling/loading.

FIG. 7 illustrates that then the second fluid channel 15 and the first fluid channel 14 may be filled with oil. Preferably, the second fluid channel 15 is filled with oil first, in order to first seal the outlets 13 of the cavities. Then, the first fluid channel 14 may also be filled with oil, so as to also seal the inlets 12 of the cavities 11.

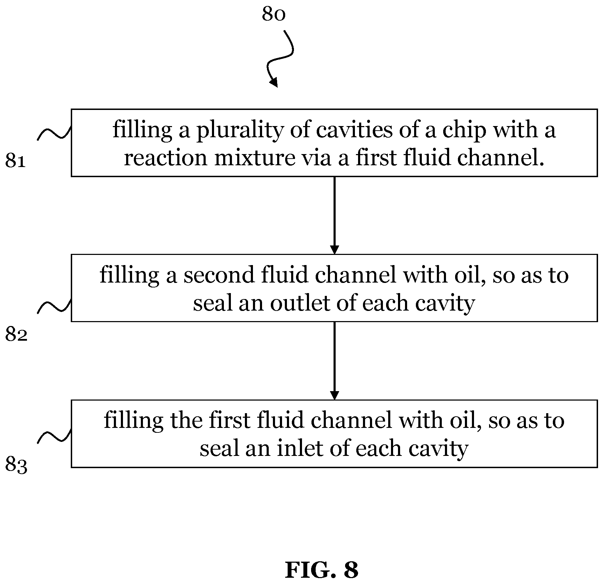

FIG. 8 shows a flow diagram of such a method 80 for operating the chip 10. The method 80 comprises the step 81 of filling the plurality of cavities 11 with the reaction mixture, then the step 82 of filling the second fluid channel with oil, and then the step 83 of filling the first fluid channel with oil.

In this way, the reaction mixture may be distributed over a plurality, e.g. thousands, of cavities 11 from the loading channel (the first fluid channel 14) using either a pressure-driven or a capillary-driven flow. The outlet structures of the cavities 11 - e.g., the narrow vertical channels at the bottom of each cavity 11 - may thereby serve as vents to the air in the cavities 11, and as a capillary valve to prevent any over-filling of the cavities 11. This reduces the sample loss significantly. After all the cavities 11 are filled with the reaction mixture, both the loading channel 14 and the sealing channel (the second fluid channel 15) may be filled with oil to separate the cavities 11 from each other and avoid cross-contamination between the cavities 11, and also to avoid evaporation of the content during thermal cycling. This also supports the reduction of sample loss.

The biochemical reaction can be performed after the cavities 11 have been filled with the reaction mixture. The biochemical reaction can be monitored optically through transparent top or bottom substrates 16, 17, and/or using the integrated pixels of the optical detector array 32 that is arranged on or in the top or bottom substrates 16, 17 of the chip 10. Performing the biochemical reaction may comprise heating the reaction mixture, for example, by using the metal absorbers or heating elements 31 as described above. Alternatively, the heat cycle may be controlled by changing the oil pressure in the sealing channel 15, wherein the oil may be used as a heating liquid. The cooling cycle may be controlled by changing the oil pressure in the sealing channel 15 so that the oil moves around in the sealing channel 15 and/or in the loading channel 14, and hence can be used as a cooling liquid. The procedure may further comprise reading out the cavities 11, for instance, by a fluorescence created in the cavities 11 through optical means. The optical means can be integrated or not integrated into the top or bottom substrates 16, 17, for instance, as the optical readout layer described before. The procedure may further comprise quantifying the optical, e.g. fluorescent, signal per cavity 11, in order to obtain digital signals of each cavity 11.

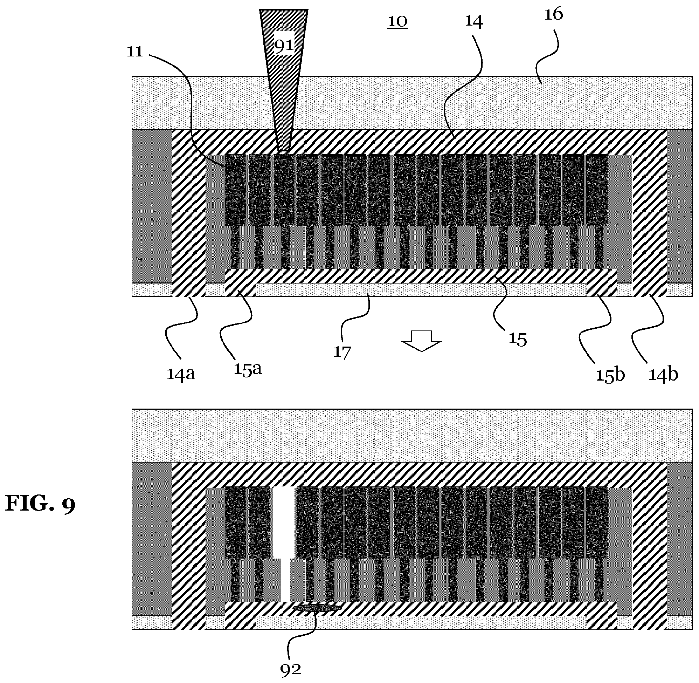

FIG. 9 illustrates a procedure of extracting content of a cavity 11 by local heating. The chip 10 shown in FIG. 9 is similar to the chip 10 in FIG. 1. Same elements in FIG. 1 and FIG. 9 are labelled with the same reference numbers and function likewise.

The content of a specific cavity 11 can particularly be extracted in the form of a droplet 92, by performing the following steps. A bubble may be nucleated in the cavity by heating up the content of the cavity 11 using a focused beam 91, for instance (to support this, a nucleation reagent can be used as ingredient of the reaction mixture). The created bubble will increase the pressure inside the cavity 11 to break the capillary barrier at the bottom of it, i.e., at the outlet 13. A droplet 92 of the cavity content will be created in the second fluid channel 15. The created droplet 92 can be manipulated by changing the oil pressure in the sealing channel 15.

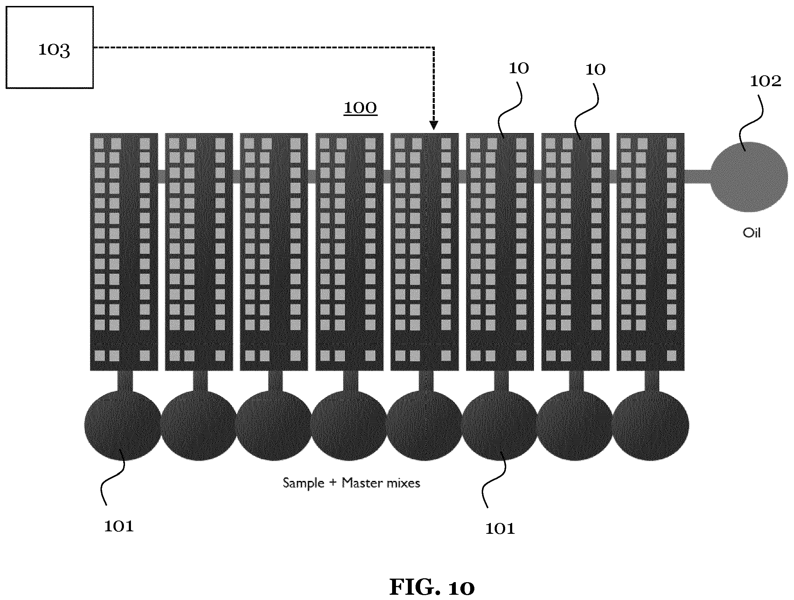

FIG. 10 shows an exemplary microfluidics system 100 according to this disclosure. The microfluidics system 100 comprises at least one chip 10 according to this disclosure, but typically multiple such chips 10 as shown. Each chip 10 may be a separate cartridge or device. The system 100 also comprises a reaction mixture reservoir 101, which is fluidically connectable to the first fluid channel 14 of the chip(s), and an oil reservoir 102, which is fluidically connectable to the first fluid channel 14 and to the second fluid channel 15 of the chip(s) 10. The system 100 may further comprise a controller 93, which is configured to control the microfluidics system 100. For instance, the controller 103 may control the filling/loading of the chip(s) 10 of the microfluidics system as described with respect to FIGs. 6-8.

Multiple samples could be loaded and mixed with a master mix on one (glass) substrate 16, 17 in the system 100, as shown in FIG. 10. The sample loading and oil inlets may be positioned below the substrate 17. The master mix and oil can also be shared between different sample inlets. An advantage of using glass panels is that mass manufacturing on panels will allow to create large substrates in which 24, 96, 384 and 1536 samples can be combined on one panel.

Multiple advantages are achieved by the solution of this disclosure. For example, a limited sample loss during loading of the reaction mixture into the cavities 11 is achieved. An automation of the sample loading procedure is possible. The solution has increased robustness, allows flexible handling of the loaded chip 10.

Also, an increased thermal control over the chip 10 and its cavities 11 is achieved, thereby enabling faster thermal cycling.

The solution of this disclosure has a higher reliability than conventional dropletbased biochemical reactions, for example, droplet PCR. Moreover, the extraction of a specific cavity content for additional analysis e.g. sequencing, is possible. The solution is also compatible with mass manufacturing on large area glass panels. In this way, millions of cavities 11 can be implemented, and an unlimited dynamic range can be achieved. Multiple samples can be processed in parallel. In addition, integrated heating using e.g. metal absorbers for fast thermal cycling of the reaction mixture can be used, and integrated cooling via oil, for example, for fast PCR cycling. Advantageously, also an in situ readout of fluorescence through optical panel detection is possible, allowing to do fast digital PCR and/or high resolution melting curves.

## Claims

1. A microfluidics chip (10) for performing a biochemical reaction in a reaction mixture, the microfluidics chip (10) comprising at least one sample unit, wherein the sample unit comprises:
a plurality of cavities (11) configured to receive the reaction mixture, wherein each cavity (11) has an inlet (12) and an outlet (13);
a first fluid channel (14) for transporting the reaction mixture to the plurality of cavities (11), wherein the first fluid channel (14) is fluidically connected to the inlet (12) of each cavity (11);
a second fluid channel (15) for transporting oil, wherein the second fluid channel (15) is connected to the outlet (13) of each cavity (11);
wherein the outlet (13) of each cavity (11) comprises a capillary barrier for the reaction mixture, the capillary barrier comprising a part of the cavity (11), the part of the cavity (11) having a smaller diameter than the rest of the cavity (11),
wherein the capillary barrier of the outlet (13) of each cavity (11) is configured such that under normal pressure conditions and operation of the chip (10) during receiving the reaction mixture by the cavities (11), surface-tension forces retain the reaction mixture within the cavity (11) and prevent the reaction mixture from passing from the cavity (11) to the second fluid channel (15), and that gas is allowed to pass from the cavity (11) to the second fluid channel (15); and
wherein the microfluidics chip (10) further comprises an optical readout layer, wherein the optical readout layer comprises an optical detector array (32), and at least one of a lenslet array (41) and a spectral-filter array (51);
wherein the spectral-filter array (51) comprises a plurality of filter pixels, wherein each cavity (11) is aligned with one filter pixel or with one group of filter pixels, wherein the filter pixel or group of filter pixels is configured to filter light from that cavity (11); and
wherein the optical detector array (32) is configured to detect light from each cavity (11) individually.

2. The chip (10) of claim 1 for performing a thermal biochemical reaction, like a polymerase chain reaction, PCR, in the reaction mixture, the chip (10) further comprising:
one or more heating elements (31) arranged and configured to heat the reaction mixture; and/or
one or more photo-thermal elements arranged and configured to heat the reaction mixture when being illuminated (81 ).

3. The chip (10) of claim 1 or 2, wherein:
the plurality of cavities (11) is an array of cavities (11); and/or
the plurality of cavities (11) is arranged between the first fluid channel (14) and the second fluid channel (15).

4. The chip (10) of one of the claims 1 to 3, wherein each cavity (11) is designed to hold a volume of the reaction mixture in a range of 0.1-100 nl.

5. The chip (10) of one of the claims 1 to 4, wherein the cavities (11) and at least one of the first fluid channel (14) and the second fluid channel (15) are transparent, so as to allow optical access.

6. The chip (10) of one of the claims 1 to 5, wherein the chip comprises a plurality of sample units, wherein each sample unit comprises an individually addressable heating element (31) and an individually addressable optical detector array (32).

7. A microfluidics system (100) comprising:
a chip (10) according to one of the claims 1 to 6;
a reaction mixture reservoir (101), which is fluidically connectable to the first fluid channel (14) of the chip (10); and
an oil reservoir (102), which is fluidically connectable to the first fluid channel (14) and to the second fluid channel (15) of the chip (10).

8. The microfluidics system (100) of claim 7, comprising a controller (103) configured to control the microfluidics system (100) to perform the method (80) of claim 9 or 10.

9. A method (80) for operating a microfluidics chip (10) or a microfluidics system (100) according to one of the claims 1 to 8, wherein the method (80) comprises:
filling (81) the plurality of cavities (11) of the chip (10) with the reaction mixture via the first fluid channel (14); then
filling (82) the second fluid channel (15) with oil, so as to seal the outlet (13) of each cavity (11); and then
filling (83) the first fluid channel (14) with oil, so as to seal the inlet (12) of each cavity.

10. The method (80) of claim 9, further comprising, after filling (82, 83) the first fluid channel (14) and the second fluid channel (15) with oil, applying a thermal heat cycle, so as to perform PCR in the reaction mixture.

## Patentansprüche

1. Mikrofluidikchip (10) zur Durchführung einer biochemischen Reaktion in einem Reaktionsgemisch, wobei der Mikrofluidikchip (10) mindestens eine Probeneinheit umfasst, wobei die Probeneinheit Folgendes umfasst:
mehrere Hohlräume (11), die dazu ausgelegt sind, das Reaktionsgemisch aufzunehmen, wobei jeder Hohlraum (11) einen Einlass (12) und einen Auslass (13) aufweist;
einen ersten Fluidkanal (14) zum Transportieren des Reaktionsgemischs zu den mehreren Hohlräumen (11), wobei der erste Fluidkanal (14) fluidisch mit dem Einlass (12) jedes Hohlraums (11) verbunden ist;
einen zweiten Fluidkanal (15) zum Transportieren von Öl, wobei der zweite Fluidkanal (15) mit dem Auslass (13) jedes Hohlraums (11) verbunden ist;
wobei der Auslass (13) jedes Hohlraums (11) eine Kapillarbarriere für das Reaktionsgemisch umfasst, wobei die Kapillarbarriere einen Teil des Hohlraums (11) umfasst, wobei der Teil des Hohlraums (11) einen kleineren Durchmesser als der Rest des Hohlraums (11) aufweist,
wobei die Kapillarbarriere des Auslasses (13) jedes Hohlraums (11) so ausgelegt ist, dass unter normalen Druckbedingungen und bei normalem Betrieb des Chips (10) während des Empfangens des Reaktionsgemischs durch die Hohlräume (11) Oberflächenspannungskräfte das Reaktionsgemisch innerhalb des Hohlraums (11) halten und verhindern, dass das Reaktionsgemisch aus dem Hohlraum (11) zu dem zweiten Fluidkanal (15) strömt; und so, dass Gas aus dem Hohlraum (11) zu dem zweiten Fluidkanal (15) strömen kann; und
wobei der Mikrofluidikchip (10) ferner eine optische Ausleseschicht umfasst, wobei die optische Ausleseschicht ein optisches Detektorarray (32) und mindestens eines aus einem Linsenarray (41) und einem Spektralfilterarray (51) umfasst;
wobei das Spektralfilterarray (51) mehrere Filterpixel umfasst, wobei jeder Hohlraum (11) mit einem Filterpixel oder mit einer Gruppe von Filterpixeln ausgerichtet ist, wobei das Filterpixel oder die Gruppe von Filterpixeln dazu ausgelegt ist, Licht aus diesem Hohlraum (11) zu filtern; und
wobei das optische Detektorarray (32) dazu ausgelegt ist, Licht von jedem Hohlraum (11) einzeln zu detektieren.

2. Chip (10) nach Anspruch 1 zur Durchführung einer thermischen biochemischen Reaktion, wie einer Polymerasekettenreaktion, PCR, in dem Reaktionsgemisch, wobei der Chip (10) ferner Folgendes umfasst:
ein oder mehrere Heizelemente (31), die zum Erwärmen des Reaktionsgemischs angeordnet und ausgelegt sind; und/oder
ein oder mehrere photothermische Elemente, die dazu angeordnet und ausgelegt sind, das Reaktionsgemisch zu erwärmen, wenn sie beleuchtet werden (81).

3. Chip (10) nach Anspruch 1 oder 2, wobei:
die mehreren Hohlräume (11) ein Array von Hohlräumen (11) sind; und/oder
die mehreren Hohlräume (11) zwischen dem ersten Fluidkanal (14) und dem zweiten Fluidkanal (15) angeordnet sind.

4. Chip (10) nach einem der Ansprüche 1 bis 3, wobei jeder Hohlraum (11) dazu ausgelegt ist, ein Volumen des Reaktionsgemischs in einem Bereich von 0,1-100 nl zu halten.

5. Chip (10) nach einem der Ansprüche 1 bis 4, wobei die Hohlräume (11) und der erste Fluidkanal (14) und/oder der zweite Fluidkanal (15) transparent sind, um einen optischen Zugang zu ermöglichen.

6. Chip (10) nach einem der Ansprüche 1 bis 5, wobei der Chip mehrere Probeneinheiten umfasst, wobei jede Probeneinheit ein individuell adressierbares Heizelement (31) und ein individuell adressierbares optisches Detektorarray (32) umfasst.

7. Mikrofluidiksystem (100), das Folgendes umfasst:
einen Chip (10) nach einem der Ansprüche 1 bis 6;
ein Reaktionsgemischreservoir (101), das mit dem ersten Fluidkanal (14) des Chips (10) fluidisch verbindbar ist; und
ein Ölreservoir (102), das mit dem ersten Fluidkanal (14) und dem zweiten Fluidkanal (15) des Chips (10) fluidisch verbindbar ist.

8. Mikrofluidiksystem (100) nach Anspruch 7, umfassend eine Steuerung (103), die dazu ausgelegt ist, das Mikrofluidiksystem (100) zu steuern, um das Verfahren (80) nach Anspruch 9 oder 10 durchzuführen.

9. Verfahren (80) zum Betreiben eines Mikrofluidikchips (10) oder eines Mikrofluidiksystems (100) nach einem der Ansprüche 1 bis 8, wobei das Verfahren (80) Folgendes umfasst:
Füllen (81) der mehreren Hohlräume (11) des Chips (10) mit dem Reaktionsgemisch über den ersten Fluidkanal (14); dann
Füllen (82) des zweiten Fluidkanals (15) mit Öl, um den Auslass (13) jedes Hohlraums (11) abzudichten; und dann Füllen (83) des ersten Fluidkanals (14) mit Öl, um den Einlass (12) jedes Hohlraums abzudichten.

10. Verfahren (80) nach Anspruch 9, ferner umfassend, nach dem Füllen (82, 83) des ersten Fluidkanals (14) und des zweiten Fluidkanals (15) mit Öl, Anwenden eines thermischen Wärmezyklus, um eine PCR in dem Reaktionsgemisch durchzuführen.

## Revendications

1. Puce microfluidique (10) pour réaliser une réaction biochimique dans un mélange réactionnel, la puce microfluidique (10) comprenant au moins une unité d'échantillon, l'unité d'échantillon comprenant :
une pluralité de cavités (11) configurées pour recevoir le mélange réactionnel, chaque cavité (11) ayant une entrée (12) et une sortie (13) ;
un premier canal à fluide (14) pour le transport du mélange réactionnel vers la pluralité de cavités (11), dans laquelle le premier canal à fluide (14) est relié fluidiquement à l'entrée (12) de chaque cavité (11) ;
un deuxième canal à fluide (15) pour le transport d'huile, le deuxième canal à fluide (15) étant relié à la sortie (13) de chaque cavité (11) ;
dans laquelle la sortie (13) de chaque cavité (11) comporte une barrière capillaire pour le mélange réactionnel, la barrière capillaire comprenant une partie de la cavité (11), la partie de la cavité (11) ayant un diamètre plus petit que le reste de la cavité (11),
dans laquelle la barrière capillaire de la sortie (13) de chaque cavité (11) est configurée de telle sorte que, dans des conditions normales de pression et de fonctionnement de la puce (10), pendant la réception du mélange réactionnel par les cavités (11), des forces de tension de surface retiennent le mélange réactionnel à l'intérieur de la cavité (11) et empêchent que le mélange réactionnel passe de la cavité (11) au second canal à fluide (15), et que du gaz puisse passer de la cavité (11) au second canal à fluide (15) ; et
dans laquelle la puce microfluidique (10) comprend en outre une couche de lecture optique, la couche de lecture optique comprenant un réseau de détecteurs optiques (32), et au moins l'un parmi un réseau de petites lentilles (41) et un réseau de filtres spectraux (51) ;
dans laquelle le réseau de filtres spectraux (51) comprend une pluralité de pixels de filtre, dans laquelle chaque cavité (11) est alignée avec un pixel de filtre ou avec un groupe de pixels de filtre, le pixel de filtre ou le groupe de pixels de filtre étant configuré pour filtrer de la lumière de cette cavité (11) ; et
dans laquelle le réseau de détecteurs optiques (32) est configuré pour détecter la lumière provenant de chaque cavité (11) individuellement.

2. Puce (10) selon la revendication 1 pour mettre en œuvre une réaction biochimique thermique, telle qu'une réaction en chaîne par polymérase, PCR, dans le mélange réactionnel, la puce (10) comprenant en outre :
un ou plusieurs éléments chauffants (31) agencés et configurés pour chauffer le mélange réactionnel ; et/ou
un ou plusieurs éléments photothermiques disposés et configurés pour chauffer le mélange réactionnel lorsqu'il est éclairé (81).

3. Puce (10) selon la revendication 1 ou 2, dans laquelle :
la pluralité de cavités (11) est un réseau de cavités (11) ; et/ou
la pluralité de cavités (11) est disposée entre le premier canal à fluide (14) et le deuxième canal à fluide (15).

4. Puce (10) selon l'une des revendications 1 à 3, dans laquelle chaque cavité (11) est conçue pour contenir un volume du mélange réactionnel dans une plage de 0,1-100 nl.

5. Puce (10) selon l'une des revendications 1 à 4, dans laquelle les cavités (11) et au moins l'un parmi le premier canal de fluide (14) et le second canal de fluide (15) sont transparents, de manière à permettre un accès optique.

6. Puce (10) selon l'une des revendications 1 à 5, dans laquelle la puce comprend une pluralité d'unités d'échantillon, dans laquelle chaque unité d'échantillon comprend un élément chauffant adressable individuellement (31) et un réseau de détecteurs optiques adressables individuellement (32).

7. Système microfluidique (100) comprenant :
une puce (10) selon l'une des revendications 1 à 6 ;
un réservoir de mélange réactionnel (101), qui peut être relié fluidiquement au premier canal de fluide (14) de la puce (10) ; et
un réservoir d'huile (102), qui peut être relié fluidiquement au premier canal de fluide (14) et au second canal de fluide (15) de la puce (10).

8. Système microfluidique (100) selon la revendication 7, comprenant un dispositif de commande (103) configuré pour commander le système microfluidique (100) pour mettre en œuvre le procédé (80) selon la revendication 9 ou 10.

9. Procédé (80) de fonctionnement d'une puce microfluidique (10) ou d'un système microfluidique (100) selon l'une des revendications 1 à 8, dans lequel le procédé (80) comprend :
remplir (81) la pluralité de cavités (11) de la puce (10) avec le mélange réactionnel via le premier canal à fluide (14) ; puis
remplir (82) le deuxième canal de liquide (15) avec de l'huile, de façon à sceller la sortie (13) de chaque cavité (11) ; puis
remplir (83) le premier canal de liquide (14) avec de l'huile de façon à sceller l'entrée (12) de chaque cavité.

10. Procédé (80) selon la revendication 9, comprenant en outre, après le remplissage (82, 83) du premier canal de fluide (14) et du second canal de fluide (15) avec de l'huile, l'application d'un cycle thermique de manière à effectuer une PCR dans le mélange réactionnel.
